(19) Europäisches Patentamt
European Patent Office
Office européen des brevets

(11) Publication number: 0 163 294 A2

# (12) EUROPEAN PATENT APPLICATION

(21) Application number: 85106586.2

(22) Date of filing: 29.05.85

(51) Int. Cl.[4]: A 61 K 49/02

(30) Priority: 01.06.84 US 616394

(43) Date of publication of application: 04.12.85 Bulletin 85/49

(84) Designated Contracting States: AT BE CH DE FR GB IT LI LU NL SE

(71) Applicant: E.I. DU PONT DE NEMOURS AND COMPANY
1007 Market Street
Wilmington Delaware 19898(US)

(72) Inventor: Patz, Michael Alan
100 Woodstock Street
Somerville Massachusetts 02144(US)

(74) Representative: von Kreisler, Alek, Dipl.-Chem. et al,
Deichmannhaus am Hauptbahnhof
D-5000 Köln 1(DE)

## (54) Soluble copper salt containing composition useful for forming technetium labelled radiodiagnostic agents.

(57) The present invention relates to compositions for making technetium-labelled radiodiagnostic agents and, in particular, to soluble copper salt containing compositions for reducing technetium or accelerating the reduction of technetium for labelling such radiodiagnostic agents, kits for preparing such $^{99m}$Tc-labelled radiodiagnostic agents with technetium, and methods for labelling such radiodiagnostic agents with technetium.

EP 0 163 294 A2

Docket No. 34531

# SOLUBLE COPPER SALT CONTAINING COMPOSITIONS USEFUL FOR FORMING TECHNETIUM LABELLED RADIODIAGNOSTIC AGENTS

## Field of the Invention

The present invention relates to compositions for making technetium-labelled radiodiagnostic agents and, in particular, to soluble copper salt containing compositions for reducing technetium or accelerating the reduction of technetium for labelling such radiodiagnostic agents, kits for preparing such $^{99m}Tc$-labelled radiodiagnostic agents with technetium, and methods for labelling such radiodiagnostic agents with technetium.

## Background of the Invention

Various complexes of monodentate and bidentate ligands with technetium have been made and studied. These complexes have been made in many cases for use in studies to determine the various oxidation states of technetium and for other research regarding the structure of such complexes and metal-coordination chemistry. Such studies have been reported in, for instance, Chemistry and Industry, pp. 347-8 (March 26, 1960); J. Inorg. Nucl. Chem., Vol. 28, pp 2293-96 (1966); Aust. J. Chem., 23, pp 453-61 (1970); Inorganic Chem., Vol. 16, No. 5, pp. 1041-48 (1977); J. Inorg. Nucl. Chem., Vol. 39, pp. 1090-92 (1977); and

J.C.S. Dalton, pp. 125-30 (1976).

In recent years many compounds or ligands have been labelled with technetium for use as radiodiagnostic agents.

For instance, in a presentation to the American Pharmaceutical Association, E.A. Deutsch disclosed that certain complexes of DIARS, i.e.

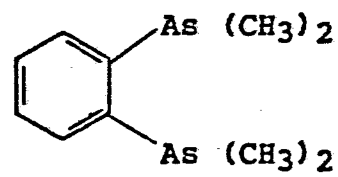

and Tc-99m, and certain complexes of DMPE, i.e. $(CH_3)_2PCH_2CH_2P(CH_3)_2$, and Tc-99m may be useful as radiodiagnostic agents for myocardial or hepatobiliary imaging. $[^{99m}Tc-(DMPE)_2Cl_2]^+$ and $[^{99m}Tc-(DIARS)_2Br_2]^+$ were prepared by Deutsch by heating in an open flask a reaction mixture containing the appropriate hydrogen halide in aqueous alcohol solution, $^{99m}Tc$-sodium pertechnetate, and ortho-phenylenebis(dimethylarsine), i.e. DIARS, or bis-(1,2-dimethylphosphino)ethane, i.e. DMPE. The reaction was reported to take about 30 minutes. The labelled complex was then purified by chromatographic methods involving ion exchange columns.

U.S. Patent No. 4,452,774 describes coordination complexes of an isonitrile ligand with various radionuclides including technetium. Representative isonitrile complexes

include those represented by the formula:

$$[A((CN)_xR)_yB_zB'_{z'}]^n$$

in which A is a radionuclide selected from radioactive isotopes of Tc, Ru, Co, Pt, Fe, Os, Ir, W, Re, Cr, Mo, Mn, Ni, Rh, Pd, Nb, and Ta, for example, $^{99m}Tc$, $^{99}Tc$, $^{97}Ru$, $^{51}Cr$, $^{57}Co$, $^{188}Re$ and $^{191}Os$; $(CN)_xR$ is a monodentate or polydentate isonitrile ligand bonded to the radionuclide through the carbon atom of the CN group; R is an organic radical; B and B' are independently selected from other ligands well known to those skilled in the art that result in isonitrile complexes, including solvents such as water, chloro and bromo groups, and ligands comprising one or more neutral donor atoms capable of forming bonds with said radionuclide; x and y are each independently, integers from 1 to 8; z and z' are each independently 0 or an integer from 1 to 7; with the proviso that (xy)+z+z' is less than or equal to 8; and n indicates the charge of the complex and can be 0 (neutral), or a positive or negative integer the value of which depends upon the valence state of A, and the charges on R, B and B'. Any desired counterion can be present as required by the charge on the complex with the proviso that such counterion must be pharmaceutically acceptable if the complex is to be used in vivo.

Particularly useful isonitrile ligands are the homoleptic six coordinate (hexakis) cationic complexes having the formula

$$[A(CNR)_6]^+$$

in which A is a monovalent radionuclide such as technetium, CNR

is a monodentate isonitrile ligand, and R is an organic radical. A suitable counterion is also present.

A bisamide bisthiol ($N_2S_2$) chelate that forms a complex with reduced technetium, $[^{99m}TcO(ema)]^-$, was described as a potential renal agent by Davison et al J. Nucl. Med. 20(60), 641 (1979). It was subsequently shown to be excreted into both the urine and bile in a chemically unchanged form, Jones et al J. Nucl. Med. 23(9), 801 (1982), thus fulfilling one of the requirements for a renal radiodiagnostic agent.

Such ligands are typically labelled with technetium by combining the ligand in solution with pertechnetate and a reducing agent. Generally, tin or stannous ion is used as the reducing agent for the technetium. However, other reducing agents have been utilized for this purpose.

U.S. 4,314,986 describes a method of reducing the pertechnetate in $TcO_4^-$ comprises mixing together an aqueous solution of pertechnetate, e.g. the eluent from a technetium generator, metallic tin or an alloy thereof as a reducing agent for the pertechnetate, and a soluble salt of a metal below tin in the electrochemical series, e.g. copper, as an activator for the tin reducing agent. A complexing agent for the reduced technetium or a colloid stabilizer may also be included. The pH is preferably 3 to 12.

European Patent Application Publication No. 0 063 946 describes a complex of technetium-99m with an acid having the formula $HOOC-CH_2-X-CH_2-COOH$, where X is sulphur or selenium,

having glomerular filtration and tubular secretion properties which make it useful in the diagnostic technique of renography to give a dynamic representation of the functioning of the kidneys. The complex may be prepared (a) by reducing $TcO_4^-$ with a $Sn^{++}$ reducing agent in the presence of $Cu^+$ and of the acid, or (b) by reducing $TcO_4^-$ with a tin metal reducing agent in the presence of a cupric salt and of the acid.

In both U.S. 3,314,986 and European Publication No. 0 063 946, the tin or stannous salt is the reducing agent and the cuprous or cupric ion is used as a catalyst for the tin or stannous salt.

Some target-seeking ligands are not labelled as easily or efficiently with technetium using conventional labelling techniques. For instance, to obtain high yields of technetium-labelled DMPE, temperatures of about 130°C are required. Thus, new techniques for labelling ligands with technetium that facilitate or accelerate the labelling process are desirable.

## Summary of the Invention

The present invention provides a method and compositions for preparing technetium complexes for use as radiodiagnostic agents. The compositions of this invention comprise and admixture of (a) a soluble copper salt and (b) a target-seeking ligand (L), or aqueous salt thereof, having the following structure:

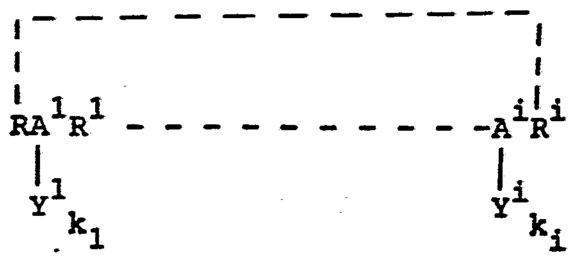

I

wherein

i is an integer from 1 to 6;

R, $R^1$, $R^2$, $R^3$, $R^4$, $R^5$ and $R^6$ are each independently selected from hydrogen or substituted or unsubstituted alkyl, aryl, alkylaryl, arylalkyl, monocycloalkyl, polycycloalkyl, heterocyclic and carbocyclic groups, and R plus $R^i$ may be taken together to form a cyclic compound or separately to form a linear compound;

$Y^1$, $Y^2$, $Y^3$, $Y^4$, $Y^5$ and $Y^6$ are independently selected from substituted or unsubstituted alkyl, aryl, alkylaryl, arylalkyl, monocycloalkyl, polycycloalkyl, heterocyclic and carbocyclic groups;

$A^1$, $A^2$, $A^3$, $A^4$, $A^5$ and $A^6$ are the same or different donor atoms, each having a free-electron pair available for complexing with Tc-99m or Tc-99; and

$k_1$, $k_2$, $k_3$, $k_4$, $k_5$ and $k_6$ are each independently zero or one; or

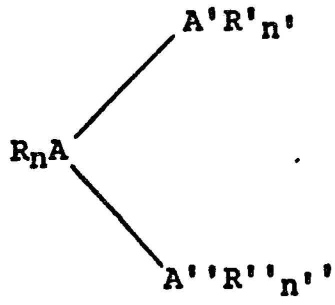

II

or:

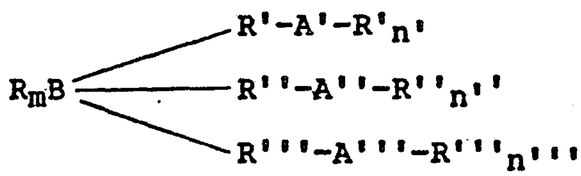

III

wherein

R, R', R" and R''' are independently selected from hydrogen or substituted or unsubstituted alkyl, aryl, alkylaryl, arylalkyl, monocylcloalkyl, polycycloalkyl, heterocyclic and carbocyclic groups;

A, A', A" and A''' are independently selected from the group of donor atoms having a pair of electrons available for complexing with Tc-99m or Tc-99;

B is an atom selected from the group of donor atoms having a pair of electrons availale for complexing with Tc-99m or Tc-99 boron, or from the elements listed in Group IV A of the periodic table (i.e. C, Si, Ge, Sn, and Pb);

m is 0 or 1; and

n', n'' and n''' are independently the integer 1 or 2.

In a preferred embodiment of this invention, the composition and kit are useful for preparing technetium labelled complexes in yield high enough to be useful for radiodiagnostic agents using ordinary water baths for heating and without the need for purification.

The compositions of this invention comprising the soluble copper salt, preferably as the cupric ion, and target-seeking ligand are preferably supplied as a kit as lyophilized solids in a pre-sterilized vial. Useful cationic technetium complexes are prepared in accord with this invention, for example, by adding $^{99m}Tc$-pertechnetate solution to the vial and heating in a water bath to obtain high yields of the desired complexes.

The R's and Y's in formulas I, II and III are preferably alkyl radicals having 1 to about 6 carbon atoms such as methyl, ethyl, etc., and the like, and aryl radicals such as benzyl, phenyl, etc., and the like.

The complexes formed from compositions and kits of this invention, when radiolabelled with technetium are useful for radiodiagnostic tests, for example, in connection with myocardial and hepatobiliary tissues.

<u>Detailed Description of the Invention</u>

The compositions and kits of the present invention can be prepared from a wide variety of monodentate and polydentate target-seeking ligands. Typical examples of such ligands

include, for instance, aryl compounds having arsenic, phosphorus, nitrogen, sulfur, oxygen, selenium, tellurium, or any combination of them, substituted ortho to each other. For example, o-phenylene compounds having the structure:

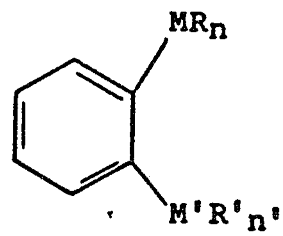

IV

in which M and M' are arsenic, phosphorus, nitrogen, sulfur, oxygen, selenium, tellurium, or any combination of them, n and n' are independently 1 or 2 depending upon the particular donor atom used for M and M', and R and R' are independently hydrogen, or an organic group, preferably an alkyl group having 1 to 6 carbon atoms, an aryl group such as phenyl, or the like, and substituted such groups. Additional examples of suitable target-seeking ligands include bidentate cis-tetraethylene ligands of the formula:

$$R'_{n'}M'-CX'_2CX_2-MR_n \qquad V$$

in which M, M', R, and R' are as defined above, n and n' are independently 1 or 2 depending upon the particular donor atom used for M and M', and X and X' are independently selected from

hydrogen, halide, or substituted or unsubstituted lower alkyl groups having 1 to about 6 carbon atoms. Further examples of suitable target-seeking ligands include those having the formula:

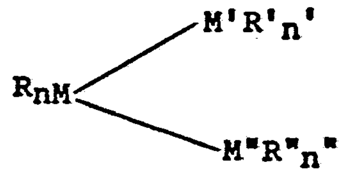

VI

where M, M', R, and R', are as defined above, M" is independently selected from arsenic, phosphorous, nitrogen, sulfur, oxygen, selenium, and tellurium, n is 0 or 1, n' and n" are independently selected from 0, 1 or 2, and R" is independently selected from hydrogen, halide or an organic radical, preferably an alkyl radical having 1 to about 6 carbon atoms, an aryl radical such as phenyl, or the like, and substituted such groups.

Particularly preferred ligands for the practice of this invention are the bis-dialkylphosphinoethanes, their substituted derivatives, and similar ligands, including, for example,
1,2-bis(dimethylphosphino)ethane,
1,2-bis(di(trifluoromethyl)phosphino)ethane,
1,2-bis(dimethylphosphino)-1,1-difluoroethane,
1,2-bis(dimethylphosphino)-1-fluoroethane,
1,2-bis(dimethylphosphino)propane,
1,2-bis(di(trifluoromethyl)phosphino)-1,1,2,2-tetrafluoroethane,
1,2-bis(di(trifluoromethyl)phosphino)propane,

2,3-bis(di(trifluoromethyl)phosphino)butane,
1,2-bis(di(trifluoromethyl)phosphino)butane,
1,3-bis(dimethylphosphino)butane,
1,3-bis(dimethylphosphino)propane,
1,3-bis(di(trifluoromethyl)phosphino)propane,
1,2-bis(dimethylphosphino)-1,1-dichloro-2,2-difluoroethane, and other similar co mpounds wherein the phosphorus is replaced by nitrogen, arsenic, sulfur, oxygen, selenium, tellurium, or any other atom having a free electron pair, and the like.

Other useful target-seeking ligands include the alkylaminobis(difluorophosphine), i.e., $RN(PF_2)_2$, ligands and the like where R is an organic group, preferably an alkyl group having 1 to about 6 carbon atoms, an aryl group as phenyl, or the like, and substituted such groups; and the o-phenylene compounds such as, for example, orthophenylenebis(diarsine), orthophenylenebis(dimethylarsine), orthophenylenebis(diamine), orthophenylenebis(dimethylamine), orthophenylenebis(diphosphine), orthophenylenebis(dimethylphosphine), and the like.

Additional target-seeking ligands suitable for use in the present invention are those described by Nozzo et al., in J. Amer. Chem. Soc., 101, p. 3683 (1979) and by Wilson et al., J. Amer. Chem. Soc., 100, p. 2269 (1978), which are hereby incorporated by reference.

Additional preferred target-seeking ligands useful in the practice of this invention include isonitrile ligands as described above. As aforementioned such isonitrile ligands

$(CN)_x$ R, where x is an integer from 1 to 8, are any monodentate or polydentate isonitrile ligands which are capable of complexing the metal atom through the carbon atom of the CN group. In the isonitrile ligand, R is an organic radical as defined for formula I above that can have additional neutral donor atoms capable of forming coordinate bonds with technetium.

Further preferred target-seeking ligands useful in the practice of this invention include tetradentate ligands or salts of such ligands having the general formula:

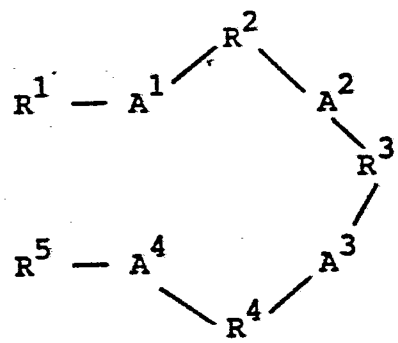

IV

where $A^1$, $A^2$, $A^3$ and $A^4$ are independently neutral donor atoms having a pair of electrons available for complexing with technetium, preferably nitrogen or sulfur atoms; and $R^1$, $R^2$, $R^3$, $R^4$ and $R^5$ are organic radicals selected from substituted and unsubstituted alkyl and aryl groups, or hydrogen where applicable. Some neutral donor atoms may require additional groups to satisfy their coordinate bond requirements not shown in formula IV above. Examples of such tetradentate ligands include,

for example,

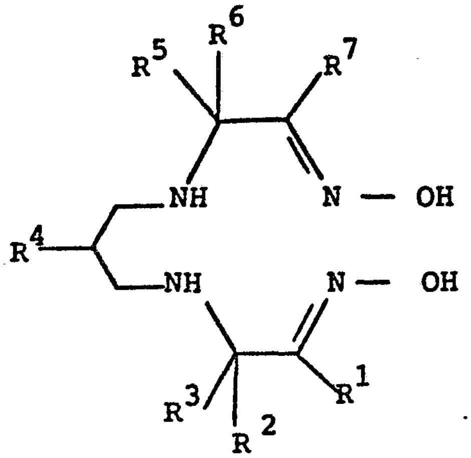

V

and bisamido, bisthio ligands such as

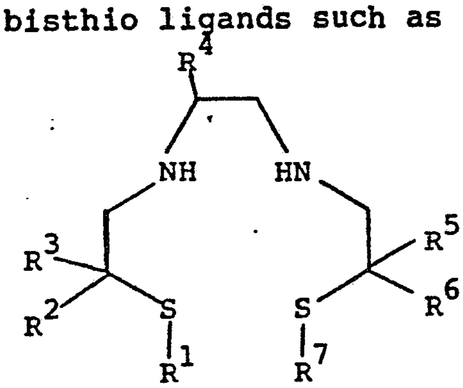

VI

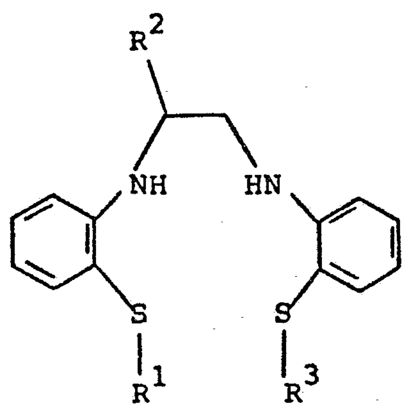

VII

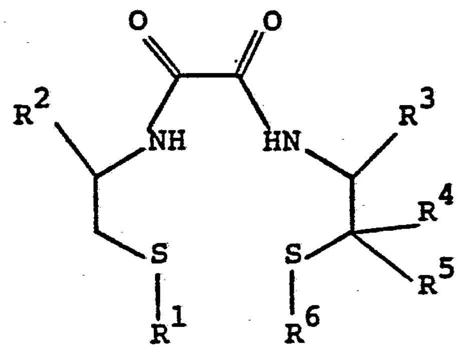

VIII

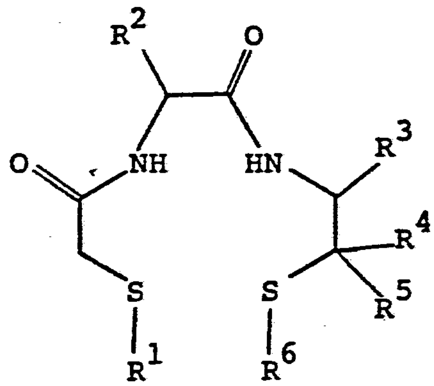

IX

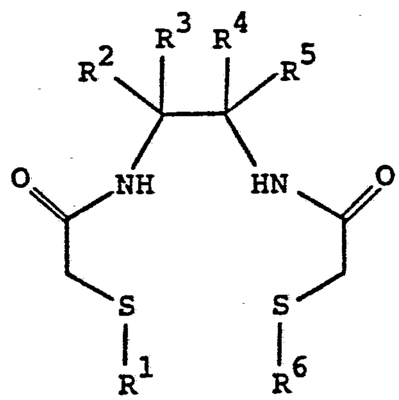

X

and similar such ligands, where the R's are independently selected from hydrogen, and substituted or unsubstituted lower alkyl groups, where the R's attached to thiol atoms are hydrogen or known thiol protecting groups.

The term "lower alkyl" when used in this application designates aliphatic saturated branched or straight chain

hydrocarbon monovalent substituents containing from 1 to 4 carbon atoms such as methyl, ethyl, isopropyl, n-propyl, n-butyl, t-butyl, etc. The term substituted lower alkyl when used herein includes alkyl groups substituted with halide, hydroxy, carboxylic acid, or carboxamide groups, etc. such as, for example, $-CH_2OH$, $-CH_2CH_2COOH$, $-CH_2CONH_2$, $-OCH_2CH_2OH$, $-OCH_2COOH$, $-OCH_2CH_2CONH_2$, etc.

Some examples of thiol protective groups are lower alkylaminocarbonyl such as ethylaminocarbonyl, lower alkanoylaminomethyl, aroylaminomethyl, t-butyl, acetamidomethyl, arylmethyl such as triphenylmethyl (trityl) and diphenylmethyl, aroyl such as benzoyl, aryloxycarbonyl such as phenoxycarbonyl, arylloweralkoxylcarbonyl, preferably arylmethoxycarbonyl such as benzyloxycarbonyl, and lower alkoxycarbonyl such as t-butoxycarbonyl. Preferred thiol protecting groups include trityl, t-butyl, diphenylmethyl, acetamidomethyl and benzoyl.

Examples of such bisamido, bisthio compounds include:

N-(2-mercaptoethyl)-(2-mercaptoacetyl)glycinamide;

N-[2-(benzoylthio)ethyl]-(2-benzoylthioacetyl)glycinamide;

N-[2-(acetamidomethylthio)ethyl]-(2-acetamidomethylthioacetyl)-glycinamide;

(2-mercaptoacetyl)glycyl-cysteine methyl ester;

(2-benzoylthioacetyl)glycyl-(S-benzoyl)cysteine methyl ester;

[2-(acetamidomethylthio)acetyl]glycyl-(S-acetamidomethyl)-cysteine methyl ester;

(2-mercaptoacetyl)glycyl-cysteine;

(2-benzoylthioacetyl)glycyl-(S-benzoyl)cysteine;

[2-(acetamidomethylthio)acetyl]glycyl-(S-acetamidomethyl)-cysteine;

(2-mercaptoacetyl)glycyl-cysteinyl-glycine methyl ester;

(2-benzoylthioacetyl)glycyl-(S-benzoyl)cysteinyl-glycine methyl ester;

[2-(acetamidomethylthio)acetyl]glycyl-(S-acetamidomethyl)-cysteinyl-glycine methyl ester;

N-(2-mercaptoethyl)-N'-(1-carbomethoxy-2-mercaptoethyl)oxamide;

N-[2-(benzoylthio)ethyl]-N'-[1-carbomethyoxy-2-(benzoylthio)-ethyl]oxamide;

N-[2-(acetamidomethylthio)ethyl]-N'-[1-carbomethoxy-2-(acet-amidomethylthio)ethyl]oxamide;

N,N'-bis(2-mercaptoethyl)oxamide;

N,N'-bis[2-(benzoylthio)ethyl]oxamide;

N,N'-bis[2-(acetamidomethylthio)ethyl]oxamide;

(R,R)N,N'-bis(1-carbomethoxy-2-mercaptoethyl)oxamide;

(R,R)N,N'-bis[1-carbomethoxy-2-(benzoylthio)ethyl]oxamide; and

(R,R)N,N'-bis[1-carbomethoxy-2-(acetamidomethylthio)ethyl]-oxamide.

The organic radicals (i.e. "R" groups) referred to in the structural formulas herein can be aliphatic or aromatic and may be substituted with a variety of groups which may or may not be charged. Among the aromatic R groups which may be present are phenyl, tolyl, xylyl, naphthyl, diphenyl and substituted aromatic groups containing such substitutents as halo, e.g., chloro,

bromo, iodo or fluoro; hydroxy, nitro, alkyl, alkoxy, etc.; among the aliphatic R groups which may be present are alkyl, preferably containing 1 to 20 carbon atoms, such as methyl, ethyl, n-propyl, isopropyl, n-butyl, isobutyl, n-hexyl, 2-ethylhexyl, dodecyl, stearyl, etc. Substituent groups may also be present in the aliphatic groups, including among others the same substituent groups as those listed above for aromatic groups.

In general, it is desirable to achieve the characteristics of the labelled complex without the need for substituent groups. Thus, it is preferred for the sake of simplicity to employ unsubstituted hydrocarbon groups as the R groups whenever possible. However, the lipophilic characteristics of the complex can be further varied by varying the R groups to adapt the complex for labelling selected materials, for membrane transport such as for the blood-brain barrier, or for imaging selected organs and dynamic processes related to their function.

Any donor element can be used in the target-seeking ligand in accord with this invention provided that it is a neutral donor atom having a free-electron pair available for accepting a proton to provide a charged ligand and further provided that it has the capability of complexing with technetium (Tc-99 or Tc-99m). Suitable such elements include, for instance, phosphorous (P), arsenic (As), nitrogen (N), oxygen (O), sulfur (S), antimony (Sb), selenium (Se), tellurium (Te), and the like.

Although technetium complexes prepared in accord with

this invention can be neutral, or positively or negatively charged, the class of lipophilic cationic complexes is presently preferred.

Preferably, the complexes prepared in accord with this invention are kinetically inert, and hence stable products. However, the complexes need only be sufficiently stable for the intended use.

Any desired counterion may also be present in the composition, such as , in the case of cationic complexes, chloride, fluoride, bromide, iodide, hydroxide, sulfate or bisulfate, dihydrogen phosphate, fluoroborate, hexafluorophosphate, etc.

The copper can be supplied by any soluble copper salt having a pharmaceutically acceptable anion, but preferably is in the form of a cupric salt. The sulfate and chloride salts are preferred.

The compositions of this invention are useful for preparing radiodiagnostic agents wherein the target-seeking ligand is labelled with technetium. Labelling is accomplished by mixing a suitable quantity of $^{99m}$Tc-pertechnetate in solution with the cupric ion and target-seeking ligand, and heating the admixture for a suitable length of time at a temperature attainable with a constant temperature water bath. Preferably, the heating step is performed at 100°C for thirty minutes or less. An aqueous physiological saline solution is typically the solution of choice for labelling the target-seeking ligand

because it is readily administered to the patient.

During labelling the pH of the solution is desirably less than 3. Preferably, the pH is less than 2.2.

It has been found that the addition of stannous ion to the composition can increase the yield of technetium complex up to about 10%. In addition, the presence of ascorbic acid in the composition significantly increases the yield of the technetium complex at higher pH levels.

The compositions of this invention are preferably contained in a kit, such as a presterilized vial. The presterilized vial, such as a glass vial, containing the compositions of this invention is ready for use for preparing cationic technetium complexes for radiodiagnostic use. More preferably, the compositions are lyophilized in such kits to increase storage stability of the compositions. In such lyophilized kits, the target-seeking ligand is generally present as a water-soluble acid salt of the ligand.

The lyophilized kits are used by reconsituting with a suitable quantity of $^{99m}$Tc-pertechnetate in saline solution. The reconstituted compositions are then placed in a constant temperature water bath for a sufficient time to form labelled technetium complexes with the target-seeking ligand. Preferably, the reaction time is about 30 minutes or less at a temperature of about 100°C.

In order to obtain high quality images the yield of radioactive labelled cationic technetium complex should

preferably be greater than 70% after reconstituting the lyophilized mixture and labelling. Lower yields will result in poorer image quality and undesirable purification steps will be required to produce high quality images.

This invention will be further illustrated by the examples that follow:

Preparation of 1,2-Bis(dimethylphosphino)ethane bis-bisulfate, i.e. $DMPEH_2^{2+}.2HSO^-_4$ or $DMPE \cdot 2H_2SO_4$

Dissolve 470 mg of DMPE in 10 ml of ethanol in a 50 ml round-bottomed flask maintained under a nitrogen atmosphere. From a glass syringe, add, with stirring, 0.34 ml of concentrated sulfuric acid. After 10 minutes, filter the precipitate and recrystallize it from 10 ml. of methanol. Filter and dry under vacuum. 920 mg of a crystalline solid is obtained, which melts at 135-136.5°C. Structure and purity of the compound was confirmed by its infra-red and nuclear magnetic resonance spectra and elemental analysis.

Comparative Example A

Dissolve 50 mg of $DMPE \cdot 2H_2SO_4$ in about 35 mls of deoxygenated physiological saline. With stirring adjust the pH to 1.0 with 3N $H_2SO_4$. Add deoxygenated saline drop-wise, to a solution weight of 40 grams. Dispense 0.8 mls of the solution

into each of several 10 cc vials that had been sealed and purged with $N_2$. Each vial is called a "kit" herein.

Dispense into each kit (i.e. vial) 0.2 mls of physiological saline containing approximately 25 mCi of $^{99m}TcO_4^-$. Place the vials in a common water bath, preheated and a constant boil; heat for 15 minutes. TLC analysis shows a yield of 2-5% of $[^{99m}TcCl_2DMPE]^+$.

Comparative Example B (Conventional Stannous Reducing Agent)

Dissolve 125 mg of $DMPE \cdot 2H_2SO_4$ and 12.5 mg of $SnCl_2 \cdot 2H_2O$ into about 95 mls of physiological saline. With stirring adjust the pH to 1.0 with 3N $H_2SO_4$. Add deoxygenated saline drop-wise until a solution weight of 100 grams. Dispense 0.8 mls of the solution into each of several 10 cc vials as in Example A above.

Dispense into each kit 0.2 mls of physiological saline containing approximately 25 mCi of $^{99m}TcO_4^-$. Place the vials in a common hot water bath, preheated and on a constant boil, heat for 15 minutes. TLC analysis shows a yield of about 10-15% of $[^{99m}TcCl_2DMPE]^+$.

Example 1

Dissolve 125 mg of $DMPE \cdot 2H_2SO_4$ and 12.5 mg of $CuSO_4 \cdot 5H_2O$ into about 95 mls of saline. With stirring adjust the pH to 1.0 with 3N $H_2SO_4$. Add deoxygenated saline drop-wise until a solution weight of 100 grams. Dispense 0.8 mls of the solution into each of several 10 cc vials (that had been sealed with a

teflon-coated stopper, crimp sealed and purged with $N_2$ for several minutes).

Dispense into each kit 0.2 mls of physiological saline containing approximately 25 mCi of $^{99m}TcO_4^-$. Place with vials in a common hot water bath, preheated and on a constant boil, heat for 15 minutes. TLC analysis shows a yield of about 95% of $[^{99m}TcCl_2DMPE]^+$.

Example 2

Dissolve 6.25 mg of $SnCl_2 \cdot 2H_2O$ in 1.0 mls of 3N $H_2SO_4$. Add 45 mls of deoxygenated physiological saline (0.15 Molar). With stirring add 62.5 mg of $DMPE \cdot 2H_2SO_4$ and 6.25 mg of $CuSO_4 \cdot 5H_2O$. Adjust the pH to 1.0 with 3N $H_2SO_4$. Add deoxygenated saline drop-wise to a solution weight of 50 grams. Dispense 0.8 mls of the solution into each of several 10 cc vials, (that had been sealed with a teflon-coated stopper, crimp sealed and purged with $N_2$ for several minutes.)

Dispense into each kit 0.2 mls of physiological saline containing approximately 25 mCi of $^{99m}TcO_4^-$. Place the vials in a common hot water bath, preheated and on a constant boil, heat for 15 minutes. TLC analysis shows a yield of 95 to 100% of $[^{99m}TcCl_2DMPE]^+$.

Example 3

Dissolve 4.0 mg $CuSO_4 \cdot 5H_2O$, 40.0 mg $DMPE \cdot 2H_2SO_4$, 4.0 mg $SnCl_2 \cdot 2H_2O$ and 800 mg Mannitol in about 35 mls of low-oxygen saline. With stirring adjust the pH to 1.0 with 3N $H_2SO_4$. Add

low-oxygen saline drop-wise until a solution weight of 40 grams. Dispense 1.0 mls of this solution into each of several 10 cc vials. Lyophilize these vials and stopper under $N_2$. Reconstitute each vial with 1.0 mls of physiological saline containing about 25 mCi of $^{99m}TcO_4^-$. Heat in a water bath for 15 minutes at 100°C. Thin layer chromatography (TLC) analysis show yields consistently greater than 95% of $[^{99m}TcCl_2DMPE]^+$.

Example 4

Dissolve 6.3 mg of $SnCl_2 \cdot 2H_2O$ in 1.0 mls of 3N $H_2SO_4$. Add 45 mls of deoxygenated physiological saline (0.15 M). With stirring add 625.0 mg of DMPE• $H_2SO_4$, 6.3 mg of $CuSO_4 \cdot 5H_2O$ and 625 mg ascorbic acid. Adjust the pH to 1.0 with 3N $H_2SO_4$. Add deoxygenated saline drop-wise, to a solution weight of 50 grams. Dispense 0.8 mls of this solution into each of several 10 cc vials (that had been sealed with a teflon-coated stopper, crimp sealed and purged with $N_2$ for several minutes).

Dispense into each kit 0.2 mls of physiological saline containing approximately 25 mCi of $^{99m}TcO_4^-$. Place the vials in a common hot water bath, preheated and on a constant boil, heat for 15 minutes. TLC analysis shows a yield of 100% of $[^{99m}TcCl_2DMPE]^+$.

Example 5

Dissolve 3.2 mg of $SnCl_2 \cdot 2H_2O$, 3.2 mg of $CuSO_4 \cdot 5H_2O$ and 31.25 mg $DMPE \cdot 2H_2SO_4$ in about 23 mls of degassed saline. With

stirring 3.2 grams of ascorbic acid is added. The pH is then adjusted to 1.8 with 3N NaOH. Add deoxygenated saline drop-wise to a solution weight of 25 grams. Dispense several 0.8 ml aliquots into 10 cc vials that are sealed and purged with $N_2$. Label kits as performed in previous examples. TLC analysis shows greater than 95% yield of $[^{99m}TcCl_2DMPE]^+$.

Example 6

Dissolve 6.3 mg of $SnCl_2 \cdot 2H_2O$ in 1.0 mls of 3N $H_2SO_4$. Add 45 mls of deoxygenated physiological saline (0.15 Molar). With stirring add 62.5 mg of $DMPE \cdot 2H_2SO_4$, 6.3 mg of $CuSO_4 \cdot 5N_2O$ and 625.0 mg of tartaric acid. Adjust the pH to 1.0 with 3N $H_2SO_4$. Add deoxygenated saline drop-wise to a solution weight of 50 grams. Dispense 0.8 mls of the solution into each of several 10 cc vials (that had been treated as in previous examples).

Dispense into each kit 0.2 mls of physiological saline containing approximately 25 mCi of $^{99m}TcO_4^-$. Place the vials in a common water bath, preheated and on a constant boil, heat for 15 minutes. TLC analysis shows a yield of 100% of $[^{99m}TcCl_2DMPE]^+$.

Examples 7-73

For each of the following examples, each formulation was in a 150 ml flat-bottom flask. To the flask was added the required amount of each solid, then an appropriate volume of degassed saline was added to dissolve the solids and bring the

total volume to near the final volume, with stirring nitrogen gas bubbled through the solution at steady rate during the preparation of bulk formulations. The pH was then adjusted with either 3N $H_2SO_4$ or 2N NaOH to the desired pH. Volume of the bulk solution was finally adjusted gravimetrically by addition of saline.

Several 0.8 ml aliquots of the bulk solution were dispensed into 10 cc vials, crimp-sealed and flushed with nitrogen. [NOTE: This volume was chosen for the kit so that addition of 0.2 ml of Tc-generator eluate would result in a 1.0 ml final volume of solution. The bulk solution was made more concentrated to compensate for this dilution.]

After reconstitution with 0.2 ml $^{99m}TcO_4^-$ eluate, each kit or vial was heated in a boiling water bath (~ 100°C) for 15 minutes unless indicated otherwise.

A. In Examples 7-16 the amount of $DMPE{\cdot}2H_2SO_4$ was varied as indicated. Otherwise, each kit or vial contained 100 ug $CuSO_4{\cdot}5H_2O$ and 100 ug $SnCl_2{\cdot}2H_2O$ at a pH of 1.0.

| Example | Amount of DMPE | Yield of $[TcCl_2(dmpe)_2]^+$ |
|---|---|---|
| 7 | 1,000 ug | 100/100* |
| 8 | 500 ug | 100/94 |
| 9 | 350 ug | 91.9 |
| 10 | 346 ug | 97.3/100 |
| 11 | 315 ug | 83.7/83.2 |
| 12 | 275 ug | 94.1/93.2 |
| 13 | 250 ug | 84.0/86.0 |
| 14 | 208 ug | 9.0/0 |
| 15 | 173 ug | 0/0 |
| 16 | 100 ug | 0/0 |

*Two values shown indicates that the measurement was repeated.

B. In Example 17-22 the amount of $DMPE \cdot 2H_2SO_4$ was varied as indicated. Otherwise, each kit or vial contained 150 ug $CuSO_4 \cdot 5H_2O$ and 100 ug $SuCl_2 \cdot 2H_2O$ at a pH of 1.0.

| Example | Amount of DMPE | % Yield of $[TcCl_2(dmpe)_2]^+$ |
|---|---|---|
| 17 | 1,000 ug | 100/100 (90.8/94.2)* |
| 18 | 467 ug | 99.2/99.2 |
| 19 | 415 ug | 100/100 (93.1/95.5) |
| 20 | 390 ug | 99.3/98.1 (93.9/99.1) |
| 21 | 380 ug | 100/97.6 (96.3/99.2) |
| 22 | 350 ug | 0/0 |

*Values in parenthesis indicate that a second vial was labelled and measured.

C. In Examples 23-28 the amount of $CuSO_4 \cdot 5H_2O$ was varied as indicated. Otherwise, each kit or vial contained 1000 ug $DMPE \cdot 2H_2SO_4$ and 100 ug $SnCl_2 \cdot 2H_2O$ at a pH of 1.0.

| Example | Amount of $CuSO_4 \cdot 5H_2O$ | % Yield of $[TcCl_2(dmpe)_2]^+$ |
|---|---|---|
| 23 | 1,000 ug | 0/0 |
| 24 | 300 ug | 100/100 (100/98.9) |
| 25 | 200 ug | 100/100 |
| 26 | 150 ug | 100/100 |
| 27 | 100 ug | 100/100 |
| 28 | 50 ug | 95.1/86.2 |

D. In the following Examples 29-31, the reaction time was shortened to 5 minutes. Otherwise, the vials were prepared as for Examples 23-28 above.

| Example | Amount of $CuSO_4 \cdot 5H_2O$ | Reaction Time | % Yield of $[^{99m}TcCl_2(dmpe)_2]^+$ |
|---|---|---|---|
| 29 | 50 ug | 5 min. | 79.0/19.4 |
| 30 | 100 ug | 5 min. | 85.7/89.3 |
| 31 | 150 ug | 5 min. | 90.8/94.2 |

E. In the following Examples 32-38 the amount of $CuSO_4 \cdot 5H_2O$ and $SnCl_2 \cdot 2H_2O$ were varied as indicated. Otherwise, each vial or kit contained 1000 ug of $DMPE \cdot 2H_2SO_4$ at a pH of 1.0.

| Example | Amount of $CuSO_4 \cdot 5H_2O$ | Amount of $SnCl_2 \cdot 2H_2O$ | % Yield of $[^{99m}TcCl_2(dmpe)_2]^+$ |
|---|---|---|---|
| 32 | 72 | 80 | 94.4/87.4 |
| 33 | 72 | 2400 | 91.8/90.3 |
| 34 | 72 | 4000 | 97.1/93.2 |
| 35 | 100 | 100 | 100/100 |
| 36 | 100 | 1000 | 98.8/100 |
| 37 | 100 | 2500 | 97.2/97.1 |
| 38 | 160 | 2400 | 100/100 |
| 39 | 0 | 100 | 21.3/20.6 |
| 40 | 100 | 0 | 95.8/95.9 |
| 41 | 200 | 0 | 94.3/94.8 |
| 42 | 300 | 0 | 96.3/96.7 |

F. In Examples 43-52 the pH and the amount of $CuSO_4 \cdot 5H_2O$ and $SnCl_2 \cdot H_2O$ were varied as indicated. Otherwise, each vial or kit contained 1000 ug $DMPE \cdot 2H_2SO_4$.

| Example | Amount of $CuSO_4 \cdot 5H_2O$ | Amount of $SnCl_2 \cdot 2H_2O$ | pH | % Yield of $[^{99m}TcCl_2-(dmpe_2]^+$ |
|---|---|---|---|---|
| 43 | 170 | 3,000 | 0.6 | 96.3 |
| 44 | 170 | 3,000 | 1.0 | 98.3/98.8 |
| 45 | 170 | 3,000 | 1.4 | 95.0/95.4 |
| 46 | 170 | 3,000 | 1.9 | 15.4/16.1 |
| 47 | 170 | 3,000 | 2.4 | 16.8/15.7 |
| 48 | 100 | 100 | 1.0 | 100/98.6 |
| 49 | 100 | 100 | 1.2 | 67.4/69.1 |
| 50 | 100 | 100 | 1.4 | 41.6/42.4 |
| 51 | 100 | 100 | 1.6 | 35.9/39.0 |
| 52 | 100 | 100 | 1.8 | 30.7/33.1 |

G. In Examples 53-60 the pH is varied as indicated. Otherwise, each vial or kit contains 1000 ug $DMPE \cdot 2H_2SO_4$, 100 ug $CuSO_4 \cdot 5H_2O$, 100 ug $SnCl_2 \cdot 2H_2O$ and 100 mg ascorbic acid.

| Examples | pH | % Yield of $[^{99m}TcCl_2(dmpe)_2]^+$ |
|---|---|---|
| 53 | 1.2 | 100/100 |
| 54 | 1.4 | 100/100 |
| 55 | 1.6 | 88.9/90.5 |
| 56 | 1.8 | 96.1/100 |
| 57 | 2.0 | 97.2/90.7 |
| 58 | 2.2 | 85.1/82.5 |
| 59 | 2.4 | 74.8/83.1 |
| 60 | 2.6 | 69.3/74.1 |

H. In Examples 61-68 the time of reaction, the pH and the amount of $CuSO_4 \cdot 5H_2O$ and $SnCl_2 \cdot 2H_2O$ were varied as indicated. Otherwise, each vial contained 1000 ug $DMPE \cdot 2H_2SO_4$ and 20 mg mannitol.

| Example | Amount of $CuSO_4 \cdot 5H_2O$ | Amount of $SnCl_2 \cdot 2H_2O$ | pH | Reaction Time | % Yield of $[^{99m}TcCl_2\text{-}(dmpe_2]^+$ |
|---|---|---|---|---|---|
| 61 | 100 ug | 100 ug | 1.2 | 15 | 73.0/70.9 |
| 62 | 100 ug | 100 ug | 1.2 | 30 | 96.6/87.9 |
| 63 | 150 ug | 150 ug | 1.0 | 15 | 96.1/100 |
| 64 | 150 ug | 150 ug | 1.0 | 30 | 100/100 |
| 65 | 150 ug | 150 ug | 1.2 | 15 | 79.6/80.0 |
| 66 | 150 ug | 150 ug | 1.2 | 30 | 95.5/95.2 |
| 67 | 150 ug | 150 ug | 1.4 | 15 | 41.5/45.2 |
| 68 | 150 ug | 150 ug | 1.4 | 30 | 67.0/66.5 |

I. In Examples 69-73 various additives were added in the amounts indicated. Otherwise, each vial or kit contained 1000 ug $DMPE \cdot 2H_2SO_4$, 100 ug $CuSO_4 \cdot 5H_2O$, and 100 ug $SnCl_2 \cdot 2H_2O$ at a pH of 1.0.

| Example | Additive | % Yield of $[^{99m}TcCl_2(dmpe)_2]^+$ |
|---|---|---|
| 69 | 10 mg Mannitol | 100.98.4 |
| 70 | 20 mg Mannitol | 96.1/100 |
| 71 | 100 mg Mannitol | 100/100 |
| 72 | 10 mg Ascorbic Acid | 100/100 |
| 73 | 10 mg Tartaric Acid | 100/100 |

Examples 74-77

In each of Examples 74-77, each formulation was prepared as in Examples 7-73 above. The particular target-seeking ligand was varied as indicated. Otherwise each vial or kit contained 1 mg target-seeking ligand, 300 ug $CuSO_4 \cdot 5H_2O$ and 100 ug $SnCl_2 \cdot 2H_2O$ at a pH of 1.0.

| Example | Target-Seeking Ligand (L) | % Yield of $[^{99m}TcCl_2(L)_2]^+$ |
|---|---|---|
| 74 | DMPP | 73.8/76.5 |
| 75 | DIMARS | 93.1/89.0 |
| 76 | DIARS | 72.8/83.2 |
| 77 | ASP | 96.4/95.2 |

DMPP = bis- (1,3 - dimethylphosphino)-propane

DIMARS = bis- (1,2 - dimethylarsino)-ethane

ASP = 1-dimethylphosphino-3-dimethylarsino-propane

The preferred ratios of materials or the optimum amounts of each target-seeking ligand, copper salt and other materials and parameters can be readily determined for particular system in a manner similar to that illustrated above in Examples 7-73. For the ligand DMPE, it appears that a molar ratio of at least 1.8 DMPE to cupric ion is required to obtain a labelled complex under the conditions investigated.

The invention has been described in detail, including the preferred embodiments thereof. However, it will be appreciated that those skilled in the art, upon consideration of this disclosure, may make modifications and improvements within the spirit and scope of the present invention.

What is claimed is:

1. A composition for preparing technetium complexes for radiodiagnostic imaging, said composition comprising an admixture of (a) a soluble copper salt and (b) a target-seeking ligand or aqueous salt thereof, having the following structure:

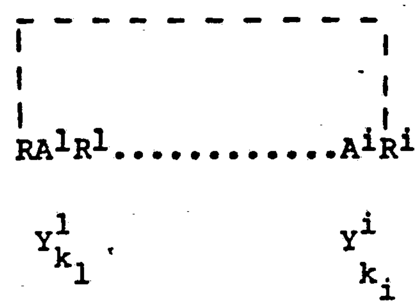

I

wherein:

i is an integer from 1 to 6;

R , $R^1$, $R^2$, $R^3$, $R^4$, $R^5$ and $R^6$ are each independently selected from hydrogen or substituted or unsubstituted alkyl, aryl, alkylaryl, arylalkyl, monocycloalkyl, polycycloalkyl, heterocyclic and carbocyclic groups, and R plus $R^i$ may be taken together to form a cyclic compound or separately to form a linear compound;

$Y^1$, $Y^2$, $Y^3$, $Y^4$, $Y^5$ and $Y^6$ are independently selected from substituted or unsubstituted alkyl, aryl, alkylaryl, arylalkyl, monocycloalkyl, polycycloalkyl, heterocyclic and carbocyclic groups;

$A^1$, $A^2$, $A^3$, $A^4$, $A^5$ and $A^6$ are each, independently, a

neutral donor atoms having a free-electron pair available for complexing with Tc-99m or Tc-99 to form a cationic complex; and

$k_1$, $k_2$, $k_3$, $k_4$, $k_5$ and $k_6$ are each independently zero or one; or

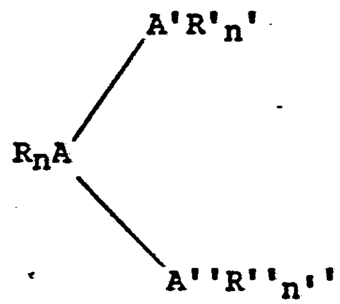

II

or

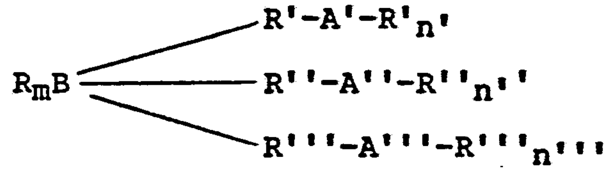

III

wherein:

R, R', R" and R''' are independently selected from hydrogen or substituted or usubstituted alkyl, aryl, alkylaryl, arylalkyl, monocylcloalkyl, polycycloalkyl, heterocyclic and carbocyclic groups;

A, A', A" and A''' are independently selected from the group of neutral donor atoms having a pair of electrons available for complexing with Tc-99m or Tc-99 to form a cationic complex;

B is an atom selected from the group of neutral donor atoms having a pair of electrons available for complexing with

Tc-99m or Tc-99 or from the elements listed in Group IV A of the periodic table;

n, n', n'' and n''' are independently the integer 1 or 2; and

m is 0 or 1.

2. The composition of Claim 1 wherein each A is selected from the group consisting of P, As, N, O, S, Sb, Se and Te.

3. The composition of Claim 1 further comprising stannous ion.

4. The composition of Claim 1 having a pH less than 3.0.

5. The composition of Claim 1 having a pH less than 2.2.

6. The composition of Claim 1 in sterile, lyophilized form in a pre-sterilized, sealed container.

7. The composition of Claim 1 wherein said target seeking ligand has the formula:

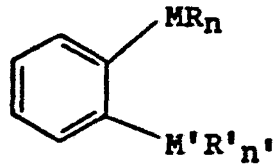

wherein M and M' are arsenic, phosphorus, nitrogen, sulfur, oxygen, selenium, tellurium, or any combination of them: R and R' are independently hydrogen, an alkyl group having from 1 to about 6 carbon atoms, or an aryl group; and n and n' are independently the integer 1 or 2.

8. The composition of Claim 1 wherein said target-seeking ligand has the formula:

$$R'_{n'}M'-CX'_2CX_2-MR_n$$

wherein M and M' are arsenic, phosphorus, nitrogen, sulfur, oxygen, selenium, tellurium, or any combination of them: R and R' are independently hydrogen, an alkyl group having from 1 to about 6 carbon atoms, or an aryl group; X and X' are independently selected from hydrogen, halide, or substituted or unsubstituted lower alkyl groups having 1 to about 6 carbon atoms; and n and n' are independently the integer 1 or 2.

9. The composition of Claim 1 wherein said target-seeking ligand has the formula:

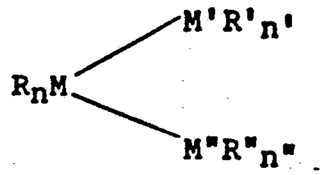

wherein M, M' and M" are independently selected from arsenic, phosphorous, nitrogen, sulfur oxygen, selenium, and tellurium; R, R' and R" are independently selected from hydrogen, halide, an alkyl group having 1 to about 6 carbon atoms, or an aryl group; n is 0 or 1; and n' and n" are independently the integer 0, 1 or 2.

10. The composition of Claim 1 wherein said target-seeking ligand has the formula:

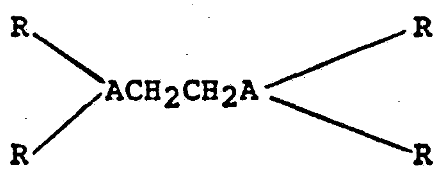

wherein A is P or As; and each R is independently H, a lower alkyl group having from 1 to about 6 carbon atoms, or phenyl.

11. The composition of Claim 1 wherein said target-seeking ligand has the formula:

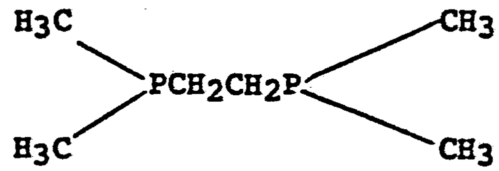

12. The composition of Claim 1 wherein said target-seeking ligand has the formula:

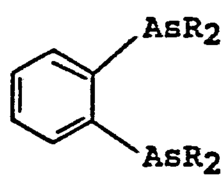

wherein R is H or a lower alkyl group having 1 to about 6 carbon atoms.

13. A method for making a complex labelled with Tc-99m for radioscintigraphic imaging, said method comprising admixing $^{99m}$Tc-pertechnetate and the composition of Claim 1 and placing the resulting admixture in a constant temperature water bath for a period of time sufficient to form such labelled complex.

14. The composition of Claim 1 wherein said target-seeking ligand is tetradentate ligand having neutral donor atoms selected from nitrogen and sulfur atoms.

15. The composition of Claim 1 wherein said

target-seeking ligand is a compound having the formula:

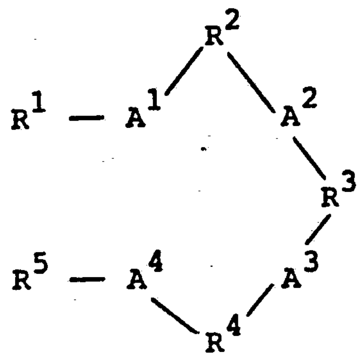

16. The composition of Claim 15 wherein each neutral donor atom is selected from nitrogen and sulfur.

17. The composition of Claim 1 wherein said target-seeking ligand is a bisamido, bisthiol compound.

18. The composition of Claim 1 wherein said target-seeking ligand has the formula:

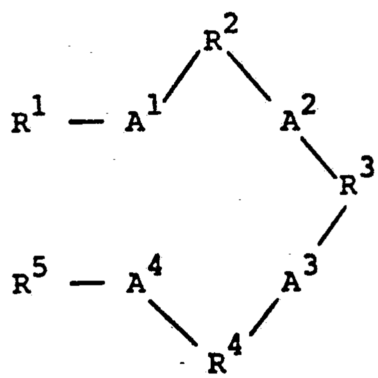

IV

19. The composition of Claim 1 wherein said target-seeking ligand has the formula:

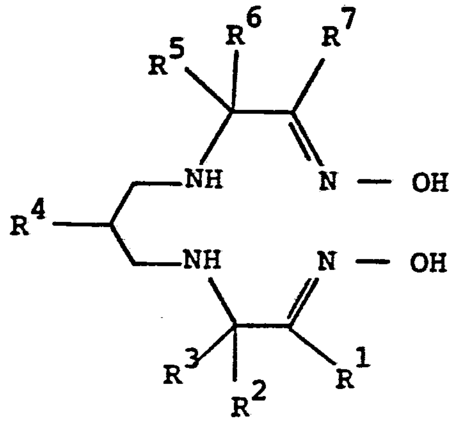

V

20. The composition of Claim 1 wherein said target-seeking ligand has the formula:

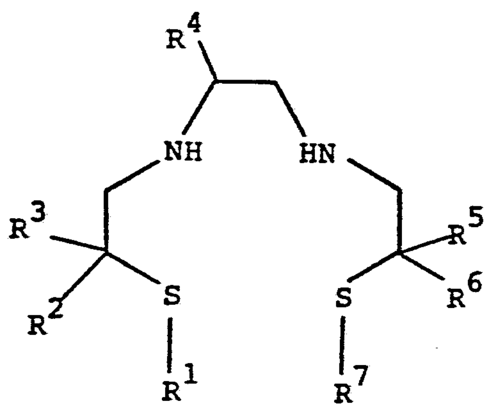

VI

21. The composition of Claim 1 wherein said target-seeking ligand has the formula:

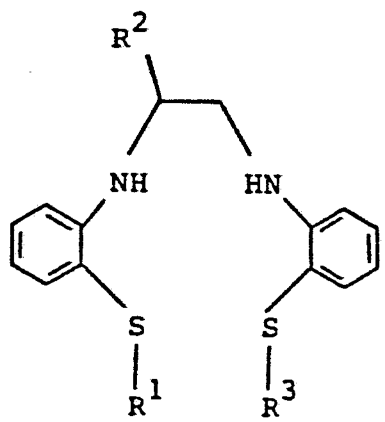

VII

22. The composition of Claim 1 wherein said target-seeking ligand has the formula:

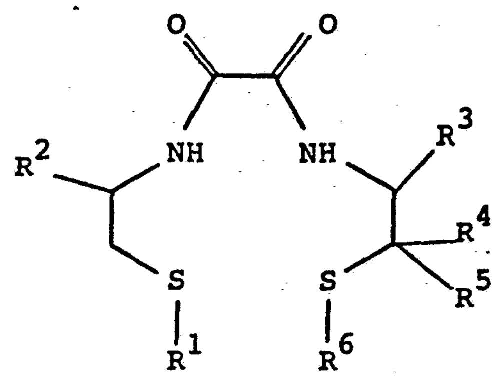

VIII

23. The composition of Claim 1 wherein said target-seeking ligand has the formula:

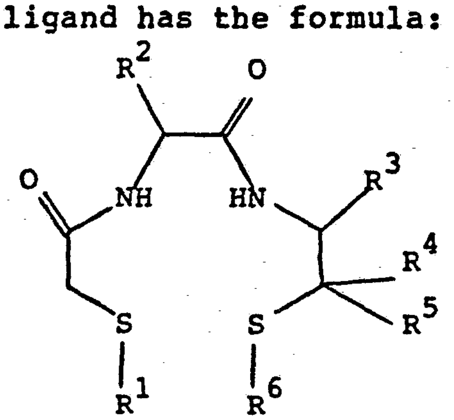

IX

24. The composition of Claim 1 wherein said target-seeking ligand has the formula:

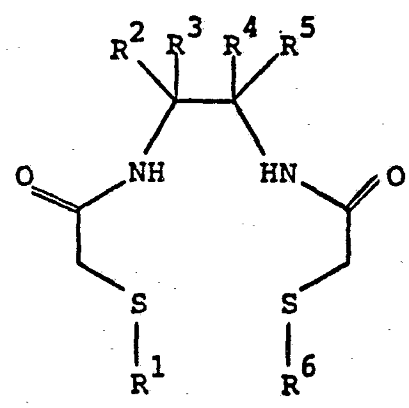

X

25. The composition of Claim 1 wherein said target-seeking ligand is an isonitrile ligand.

26. The composition of Claim 1 wherein said target-seeking ligand has the formula $(CN)_xR$ where x is an integer from 1 to 8.